(19) Europäisches Patentamt
European Patent Office
Office européen des brevets

(11) Veröffentlichungsnummer: **0 036 971**
**B1**

# (12) EUROPÄISCHE PATENTSCHRIFT

(45) Veröffentlichungstag der Patentschrift: **17.08.83**

(51) Int. Cl.³: **C 07 D 209/86**

(21) Anmeldenummer: **81101702.9**

(22) Anmeldetag: **09.03.81**

## (54) Verfahren zur Herstellung von N-Hydroxyalkylcarbazol.

(30) Priorität: **27.03.80 DE 3011808**

(43) Veröffentlichungstag der Anmeldung: **07.10.81 Patentblatt 81/40**

(45) Bekanntmachung des Hinweises auf die Patenterteilung: **17.08.83 Patentblatt 83/33**

(84) Benannte Vertragsstaaten: **BE CH DE FR GB LI**

(56) Entgegenhaltungen: **DE-A-2 354 325**
**DE-A-2 354 326**

(73) Patentinhaber: **CASSELLA Aktiengesellschaft, Hanauer Landstrasse 526, D-6000 Frankfurt am Main 61 (DE)**

(72) Erfinder: **Tappe, Horst, Dr., Ringstrasse 9, D-6057 Dietzenbach (DE)**

(74) Vertreter: **Urbach, Hans-Georg, Dr. et al, Hanauer Landstrasse 526, D-6000 Frankfurt am Main 61 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

EP 0 036 971 B1

# Verfahren zur Herstellung von N-Hydroxyalkylcarbazol

Die Erfindung betrifft ein Verfahren zur Herstellung von N-Hydroxyalkylcarbazol der Formel I

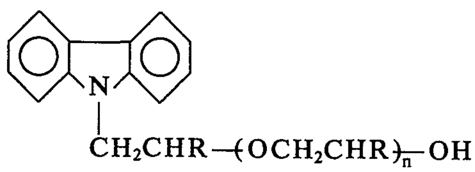

(I)

worin R = H, $CH_3$ oder $C_2H_5$ und n eine Zahl von 0 bis 4 bedeutet. Vorzugsweise bedeutet R = H.

N-$\beta$-Hydroxyethylcarbazol ist ein wichtiges Ausgangsprodukt, z. B. für die Polymerisation von Vinylcarbazol zu Polyvinylcarbazol. Polyvinylcarbazol besitzt dann besonders gute Eigenschaften als Photoleiter bei seiner Anwendung in der Elektrophotographie, wenn das für die Polymerisation benutzte Vinylcarbazol durch Dehydratisation von N-$\beta$-Hydroxyethylcarbazol erhalten worden ist. Ferner ist N-$\beta$-Hydroxyethylcarbazol als Kupplungskomponente für die Herstellung von Azofarbstoffen geeignet (US-PS 3 787 178).

Es sind bereits viele Verfahren zur Herstellung von N-$\beta$-Hydroxyehtylcarbazol durch Oxethylierung von Carbazol mit Ethylenoxid oder Ethylenchlorhydrin beschrieben worden. Diese Umsetzungen erfordern in Ketonen, wie z. B. Aceton als Lösungsmittel häufig lange Reaktionszeiten von z. B. 26 bis 30 Stunden (GB-PS 620 733; J. Amer. Chem. Soc. 70, 3019 (1948)). Die Isolation des Produktes erfordert dabei oft aufwendige Extraktions- und Kristallisationsverfahren, wobei das Lösungsmittel häufig vernichtet wird. In Abwesenheit von Ketonen verläuft die Oxethylierung von Carbazol nur mit minimalen Ausbeuten von z. B. 2% (C.A. 63, 565 (1965)). Bei anderen Verfahren müssen als Lösungsmittel hochsiedende Ether (vgl. CA 81, 25 550 (1974)) verwendet oder die isolierten Alkalisalze des Carbazols eingesetzt werden (DE-OS 2 354 326), die ihrerseits zuvor in getrennten Verfahren hergestellt werden müssen, z. B. durch Erhitzen von Carbazol in einem organischen Lösungsmittel, das ein azeotrop siedendes Gemisch mit Wasser bildet und unter Abtrennung des bei der Umsetzung gebildeten Wassers (DE-OS 2 354 325).

Bei anderen Verfahren wird mit teuren Lösungsmitteln, wie Dimethylsulfoxid (FR-PS 1 527 778) gearbeitet, die anschließend noch mit Wasser verdünnt werden und so verloren sind oder nur schwer und aufwendig regeneriert werden können.

Es bestand daher das dringende Bedürfnis nach einem einfachen Verfahren zur Oxalkylierung von Carbazol.

Überraschenderweise wurde nun gefunden, daß man Carbazol sehr einfach in guten Ausbeuten entgegen den Lehren von CA 63, 565 (1965) oxalkylieren kann. Das erfindungsgemäße Verfahren zur Herstellung von N-Hydroxyalkylcarbazol der Formel I ist dadurch gekennzeichnet, daß man Carbazol in einem mit Wasser nicht mischbaren organischen Lösungsmittel bei Temperaturen von 20 bis 150° C in Gegenwart eines Alkalimetallhydroxids oder Alkalimetallhydrids und eines Phasentransferkatalysators mit einem Epoxid der Formel II oder Chlorhydrin der Formel III

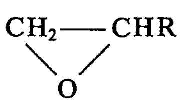

(II)

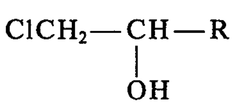

(III)

umsetzt.

Die Durchführung des erfindungsgemäßen Verfahrens erfolgt in der Weise, daß man die Reaktanten unter den oben angegebenen erfindungsgemäßen Reaktionsbedingungen in einem Mehrphasensystem, das aus einer flüssigen, nicht wäßrigen und einer oder mehreren fester Phasen besteht, zusammen reagieren läßt. So ist es beispielsweise im Prinzip möglich, alle erforderlichen Komponenten der Reaktionsmischung von Anfang an in den erforderlichen Mengenverhältnissen zu mischen, sofern durch geeignete Maßnahmen für einen störungsfreien Ablauf der Reaktion gesorgt wird, d. h. daß in diesem Fall für eine ausreichende Wärmeabfuhr und bei gasförmigen Ausgangsmaterialien durch einen genügend hohen Druck für eine Verflüssigung Sorge zu tragen ist.

Um von vornherein Schwierigkeiten bei der Reaktionsführung zu vermeiden, wird man in der Regel eine Ausführungsform des erfindungsgemäßen Verfahrens wählen, bei der Carbazol gelöst oder ungelöst und die Base ungelöst in einem System aus Lösungsmittel und Phasentransferkatalysator vorgelegt und das Epoxid oder Chlorhydrin anschließend zudosiert wird.

Durch Variation des Molverhältnisses Carbazol : Epoxid bzw. Chlorhydrin kann man Verbindungen mit verschiedenen Werten für n erhalten. Bei den in der nachfolgenden Tabelle angegebenen

Molverhältnissen Carbazol : Epoxid (Epoxide der Formel II, insbesondere das Ethylenoxid, werden vorzugsweise verwendet) werden theoretisch die angegebenen Werte für n in der Verbindung I erhalten:

| Molverhältnis | |
|---|---|
| Carbazol : Epoxid | n |
| 1 : 1 | 0 |
| 1 : 2 | 1 |
| 1 : 3 | 2 |
| 1 : 4 | 3 |
| 1 : 5 | 4 |

Bei der praktischen Durchführung können bei Molverhältnissen Carbazol : Epoxid (bzw. Halogenhydrin) = 1 : (>1) Gemische von Verbindungen der Formel I mit verschiedenen n entstehen, was sogar für verschiedene Anwendungszwecke, z. B. bei der Verwendung als Kupplungskomponente, von Vorteil ist.

Als mit Wasser nicht mischbare, organische Lösungsmittel werden aliphatische oder aromatische Kohlenwasserstoffe verwendet, die auch halogeniert sein können. Normalerweise werden solche Lösungsmittel verwendet, die bei Normaltemperatur flüssig sind. Auch Mischungen verschiedener Lösungsmittel können verwendet werden. Geeignete Lösungsmittel sind z. B. Pentan, Hexan, Heptan, Octan, Nonan, Decan, Petrolether, Ligroin, Leicht- und Schwerbenzin, Benzol, Alkalibenzole mit 1 bis 3 Alkyl-, insbesondere Methylresten, wie z. B. Toluol, ortho-, meta-, para-Xylol, Ethylbenzol, Diethylbenzol, Isopropylbenzol, Halogenbenzole oder Halogenalkylbenzole mit 1 bis 3 Halogenen, insbesondere Chlorsubstituenten, wie z. B. Monochlorbenzol, ortho-, meta-, para-Dichlorbenzol, ortho-, meta-, para-Chlortoluol.

Vorzugsweise werden solche Lösungsmittel oder Lösungsmittelgemische verwendet, die Siedepunkte zwischen 50 und 180°C besitzen. Als Lösungsmittel bevorzugt werden Toluol oder Monochlorbenzol. Auch eine sehr geringe Löslichkeit des Carbazols oder der verwendeten Base in dem zur Anwendung kommenden Lösungsmittel oder Lösungsmittelgemisch beeinträchtigt den glatten Verlauf des erfindungsgemäßen Verfahrens nicht nachteilig.

Als Alkalimetallhydroxid wird vorzugsweise Kalium- oder Natriumhydroxid, insbesondere in gepulverter Form, verwendet. Auch Mischungen verschiedener Alkalimetallhydroxide und/oder Alkalimetallhydride können benutzt werden. Normalerweise werden pro Mol Carbazol 1 bis 3 Mole Alkalimetallhydroxid oder Alkalimetallhydrid eingesetzt.

Die Menge des einzusetzenden Phasentransferkatalysators beträgt zweckmäßigerweise 2 bis 20 Mol-%, bezogen auf die Menge Carbazol. Vorzugsweise werden 4 bis 15 Mol-%, insbesondere 10 bis 15 Mol-% des Phasentransferkatalysators eingesetzt. Eine zusammenfassende Übersicht über geeignete Phasentransferkatalysatoren befindet sich beispielsweise in dem Buch von W. P. Weber und G. W. Gokel, Phase Transfer Catalysis in Organic Synthesis, Springer Verlag, Berlin, New York 1977. Für die Durchführung des erfindungsgemäßen Verfahrens kommen als Phasentransferkatalysatoren insbesondere quartäre Ammoniumsalze der Formel IV

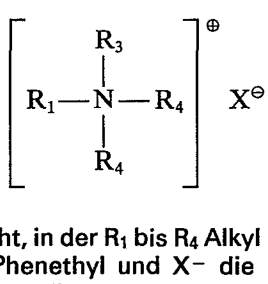

(IV)

in Betracht, in der $R_1$ bis $R_4$ Alkyl mit 1 bis 18 C-Atomen, Alkylphenyl mit 1 bis 10 C-Atomen im Alkylrest, Benzyl, Phenethyl und $X^-$ die Ionen $Cl^-$, $Br^-$, $SO_4^{--}$, $HSO_4^-$ bedeuten, ferner Kronenether und gegebenenfalls veretherte Polyethylenglykolderivate, z. B. Polyethylenglykoldimethylether mit Molgewichten von beispielsweise bis zu 600. Bevorzugt werden Kronenether. Besonders bevorzugt werden veretherte Polyethylenglykolderivate und veretherte Mischpolymerisate aus Ethylenoxid und Tetrahydrofuran.

Geeignete Phasentransferkatalysatoren der Formel IV sind beispielsweise

Di(-dodecyl)-dimethylammoniumchlorid,

Hexadecyltrimethylammoniumchlorid,
Tetrabutylammoniumchlorid,
Trioctylmethylammoniumchlorid,
Tris-decylmethylammoniumchlorid,
Trialkyl-($C_8-C_{10}$-Gemisch)-methylammoniumchlorid.

Geeignete Kronenether sind z. B.
Dicyclohexano[18]krone-6,
[18]Krone-6, [15]Krone-5, Dibenzo[18]krone-6.

Das erfindungsgemäße Verfahren wird bei Temperaturen zwischen 20 bis 180°C durchgeführt. Besonders günstig ist der Temperaturbereich von 60 bis 135° C, bevorzugt der von 80 bis 120° C.

Beispiele für Verbindungen der Formeln II und III sind Ethylenoxid, Propylenoxid und 2-Chlor-ethanol. Vorzugsweise wird Ethylenoxid verwendet.

Die Aufarbeitung der Reaktionsansätze nach beendeter Umsetzung erfolgt in an sich bekannter Weise. Zweckmäßigerweise wird das in den Reaktionsansätzen enthaltene Alkalimetallhydroxid oder Alkalimetallhydrid durch Zugabe von Wasser und Abscheiden der wäßrigen Phase abgetrennt. Das organische Lösungsmittel enthält dann nur noch das Endprodukt, das durch an sich bekannte Verfahren, wie z. B. Destillation, leicht rein gewonnen werden kann.

Das Abdestillieren des organischen Lösungsmittels kann bei Normaldruck oder vermindertem Druck oder mit Wasserdampf erfolgen.

Das erfindungsgemäße Verfahren gestattet die Herstellung von Verbindungen, die beispielsweise als Zwischenprodukte für die organische Synthese von hohem Wert sind in hohen Ausbeuten und sehr guter Reinheit auf technisch besonders einfache und ökologisch einwandfreie Weise. Es ist im Hinblick auf diese Merkmale den nächstvergleichbaren Verfahren erheblich überlegen. Insbesondere entfällt nach dem erfindungsgemäßen Verfahren die umständliche und kostspielige Herstellung des Alkalimetallsalzes des Carbazols. So ist das nach dem erfindungsgemäßen Verfahren hergestellte $\beta$-Hydroxyethylcarbazol nur von schwach gelblicher Farbe, während man nach dem bekannten mit Aceton als Lösungsmittel arbeitenden Verfahren gelbbraune bis schwarze Verbindungen erhält.

In den folgenden Ausführungsbeispielen sind alle Teile Gewichtsteile und Prozente Gewichtsprozente.

## Beispiel 1

In einen 2-Liter-Vierhalskolben gibt man 1 l Toluol, 167 g (1 Mol) Carbazol, 60 g NaOH-Pulver, 40 g Pentaethylenglykoldimethylether.

Man rührt 1 Stunde bei 110°C, kühlt auf 85°C ab und leitet unter Rühren in 1 Stunde 1,1 Mol Ethylenoxid ein. Nach beendeter Umsetzung wird auf 20° C abgekühlt, 800 ml Wasser zugegeben, die wäßrige Phase im Scheidetrichter abgetrennt, die organische Phase mit Wasser gewaschen und dann destilliert. Zunächst wird das Toluol unter Normaldruck und anschließend unter vermindertem Druck des N-$\beta$-Hydroxyethylcarbazols bei 206 bis 210° C/2,66 mbar abdestilliert.

Ausbeute: 194 g N-$\beta$-Hydroxyethylcarbazol als schwach gelbes Produkt.

## Beispiel 2

Beispiel 1 wird wiederholt, jedoch werden 30 g eines Mischpolyglykol-dimethylethers, hergestellt aus Tetrahydrofuran, Ethylenoxid und Methylchlorid (pro Mol werden 5 Mol Ethylenoxid, 1 Mol Tetrahydrofuran und 2 Mol Methylchlorid verwendet) eingesetzt.

Ausbeute: 187 g N-$\beta$-Hydroxyethylcarbazol.

## Patentansprüche

1. Verfahren zur Herstellung von N-Hydroxyalkylcarbazol der Formel I

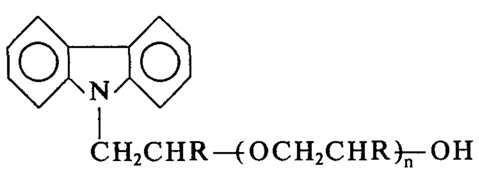

(I)

worin R = H, $CH_3$ oder $C_2H_5$ und n eine Zahl von 0 bis 4 bedeutet, dadurch gekennzeichnet, daß man Carbazol in einem mit Wasser nicht mischbaren organischen Lösungsmittel bei Temperaturen von 20 bis 150°C in Gegenwart eines Alkalimetallhydroxids oder Alkalimetallhydrids und eines Phasentransferkatalysators mit einem Epoxid der Formel II oder Chlorhydrin der Formel III

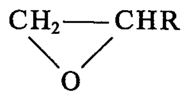

(II)

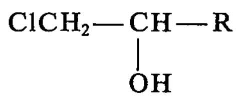

(III)

umsetzt.

2. Verfahren nach Anspruch 1, dadurch gekennzeichnet, daß man als Alkalimetallhydroxid Natrium- oder Kaliumhydroxid verwendet.

3. Verfahren nach den Ansprüchen 1 oder 2, dadurch gekennzeichnet, daß man, bezogen auf Carbazol, 2 bis 20 Mol-%, vorzugsweise 4 bis 15 Mol-%, des Phasentransferkatalysators einsetzt.

4. Verfahren nach einem oder mehreren der Ansprüche 1 bis 3, dadurch gekennzeichnet, daß man, bezogen auf Carbazol, 10 bis 15 Mol-% des Phasentransferkatalysators einsetzt.

5. Verfahren nach den Ansprüchen 1 bis 4, dadurch gekennzeichnet, daß man das Verfahren bei 60 bis 120° C durchführt.

6. Verfahren nach einem oder mehreren der Ansprüche 1 bis 5, dadurch gekennzeichnet, daß man das Verfahren bei 80 bis 120° C durchführt.

7. Verfahren nach einem oder mehreren der Ansprüche 1 bis 6, dadurch gekennzeichnet, daß man als Phasentransferkatalysator ein quartäres Ammoniumsalz der Formel IV

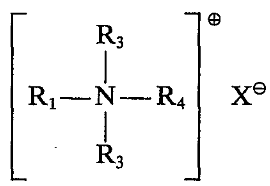

(IV)

worin $R_1$ bis $R_4$ Alkyl mit 1 bis 18 C-Atomen, Alkylphenyl mit 1 bis 10 C-Atomen im Alkylrest, Benzyl, Phenethyl und $X^-$ die Ionen $Cl^-$, $Br^-$, $SO_4^{--}$, $HSO_4^-$ bedeuten, verwendet.

8. Verfahren nach einem oder mehreren der Ansprüche 1 bis 7, dadurch gekennzeichnet, daß man als Phasentransferkatalysator Dicyclohexano[18]krone-6, [18]Krone-6, [15]Krone-5 oder Dibenzo[18]krone-6 verwendet.

9. Verfahren nach einem oder mehreren der Ansprüche 1 bis 8, dadurch gekennzeichnet, daß man als Phasentransferkatalysator einen Polyethylenglykol-dimethylether mit einem Molgewicht bis 600 verwendet.

10. Verfahren nach einem oder mehreren der Ansprüche 1 bis 9, dadurch gekennzeichnet, daß man als Lösungsmittel Petrolether, Ligroin, Benzol, Toluol, Chlorbenzol, Dichlorbenzol oder Chlortoluol verwendet.

**Claims**

1. Process for the manufacture of an N-hydroxyalkylcarbazole of the formula I

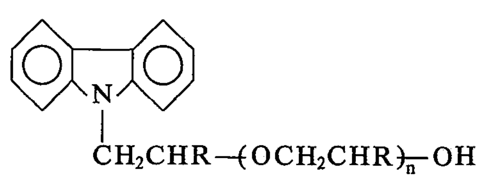

(I)

wherein R denotes H, $CH_3$ or $C_2H_5$ and n denotes a number from 0 to 4, characterised in that carbozole is reacted with an epoxide of the formula II or a chlorohydrin of the formula III

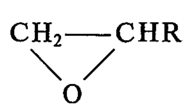

(II)

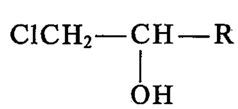

(III)

at temperatures from 20 to 150° C in the presence of an alkali metal hydroxide or an alkali metal hydride and a phase transfer catalyst, in an organic solvent which is immiscible with water.

2. Process according to Claim 1, characterised in that the alkali metal hydroxide used is sodium hydroxide or potassium hydroxide.

3. Process according to Claim 1 or 2, characterised in that 2 to 20 mol %, preferably 4 to 15 mol %, relative to carbazole, of the phase transfer catalyst are employed.

4. Process according to one or several of Claims 1 to 3, characterised in that 10 to 15 mol %, relative to carbazole, of the phase transfer catalyst are employed.

5. Process according to Claims 1 to 4, characterised in that the process is carried out at 60 to 120° C.

6. Process according to one or several of Claims 1 to 5, characterised in that the process is carried out at 60 to 120° C.

7. Process according to one or several of Claims 1 to 6, characterised in that the phase transfer catalyst used is a quaternary ammonium salt of the formula IV

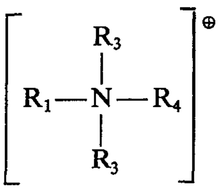

(IV)

wherein $R_1$ to $R_4$ denote alkyl having 1 to 18 C atoms, alkylphenyl having 1 to 10 C atoms in the alkyl radical, benzyl, phenethyl and $X^-$ denotes the ions $Cl^-$, $Br^-$, $SO_4^{--}$ or $HSO_4^-$.

8. Process according to one or several of Claims 1 to 7, characterised in that the phase transfer catalyst used is dicyclohexano[18]crown-6, [18]crown-6, [15]crown-5 or dibenzo [18]crown-6.

9. Process according to one or several of Claims 1 to 8, characterised in that the phase transfer catalyst used is a polyethylene glycol dimethylether having a molecular weight of up to 600.

10. Process according to one or several of Claims 1 to 9, characterised in that the solvent used is petroleum ether, ligroin, benzene, toluene, chlorobenzene, dichlorobenzene or chlorotoluene.

**Revendications**

1. Procédé de préparation de N-(hydroxyalkyl)-carbazoles répondant à la formule I

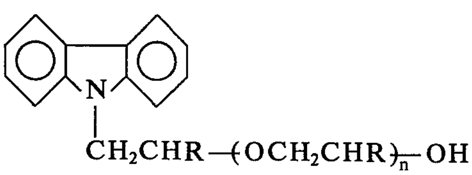

(I)

dans laquelle R représente H, $CH_3$ ou $C_2H_5$ et n désigne un nombre de 0 à 4, procédé caractérisé en ce qu'on fait réagir le carbazole dans un solvant organique non miscible à l'eau, à des températures de 20 à 150° C, en présence d'un hydroxyde de métal alcalin ou d'un hydrure de métal alcalin et d'un catalyseur à transfert de phases, avec un époxyde de formule II ou une chlorhydrine de formule III

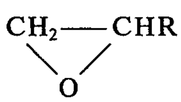

(II)

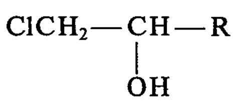

(III)

2. Procédé selon la revendication 1 caractérisé en ce qu'on utilise, comme hydroxyde de métal alcalin, l'hydroxyde de sodium ou l'hydroxyde de potassium.

3. Procédé selon l'une des revendications 1 et 2, caractérisé en ce qu'on met en jeu, par rapport au carbazole, de 2 à 20% en moles du catalyseur à transfert de phases, de préférence de 4 à 15% en moles.

4. Procédé selon l'une quelconque des revendications 1 à 3, caractérisé en ce qu'on met en jeu, par rapport au carbazole, de 10 à 15% en moles du catalyseur à transfert de phases.

5. Procédé selon l'une quelconque des revendications 1 à 4, caractérisé en ce qu'on opère à une température de 60 à 120° C.

6. Procédé selon l'une quelconque des revendications 1 à 5, caractérisé en ce qu'on opère à une température 80 à 120° C.

7. Procédé selon l'une quelconque des revendications 1 à 6, caractérisé en ce qu'on utilise, comme catalyseur à transfert de phases, un sel d'ammonium quaternaire répondant à la formule IV

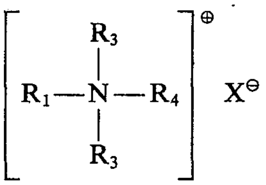

(IV)

dans laquelle $R_1$ à $R_4$ représentent chacun un alkyle contenant de 1 à 18 atomes de carbone, un alkylphényle dont la partie alkylique contient de 1 à 10 atomes de carbone, un benzyle ou un phényléthyle, et $X^-$ représente un ion $Cl^-$, $Br^-$, $SO_4^{--}$, ou $HSO_4^-$.

8. Procédé selon l'une quelconque des revendications 1 à 7, caractérisé en ce qu'on utilise, comme catalyseur à transfert de phases, la dicyclohexano[18]couronne-6, la [18]couronne-6, la [15]couronne-5 ou la dibenzo[18]couronne-6.

9. Procédé selon l'une quelconque des revendications 1 à 8, caractérisé en ce qu'on utilise, comme catalyseur à transfert de phases, un éther diméthylique de polyéthylène-glycol dont le poids moléculaire peut aller jusqu'à 600.

10. Procédé selon l'une quelconque des revendications 1 à 9, caractérisé en ce qu'on utilise, comme solvant, l'éther de pétrole, la ligroine, le benzène, le toluène, le chlorobenzène, un dichlorobenzène ou un chlorotoluène.